# FASCICULE DE BREVET EUROPEEN

(11) **EP 3 765 067 B1**
(45) Date de publication et mention de la délivrance du brevet: **16.04.2025**
(21) Numéro de dépôt: 19713118.8
(22) Date de dépôt: 13.03.2019
(51) Int. Cl.: A61K 39/00, A61P 35/00

(54) **VACCINS AUTOLOGUES CONTRE LE CANCER**
AUTOLOGE KREBSVAKZINE
AUTOLOGOUS CANCER VACCINES

(30) Priorité: 14.03.2018 FR 1852197
(43) Date de publication de la demande: 20.01.2021
(73) Titulaire: Hastim, 31300 Toulouse (FR)
(72) Inventeur: FRAYSSINET, Patrick, 31470 Saint Lys (FR); ROUQUET, Nicole, 31400 Toulouse (FR)
(74) Mandataire: Plasseraud IP
(86) Numéro de dépôt international: PCT/FR2019/050537
(87) Numéro de publication internationale: WO 2019/175500

(56) Documents cités:
- EP-A1- 2 564 867
- WO-A1-2014/184453
- WO-A2-2006/122914
- MARCONATO L ET AL: "Enhanced therapeutic effect of APAVAC immunotherapy in combination with dose-intense chemotherapy in dogs with advanced indolent B-cell lymphoma", VACCINE, vol. 33, no. 39, 18 August 2015 (2015-08-18), pages 5080 - 5086, XP029272461, ISSN: 0264-410X, DOI: 10.1016/J.VACCINE.2015.08.017
- LAURA MARCONATO ET AL: "Randomized, Placebo-Controlled, Double-Blinded Chemoimmunotherapy Clinical Trial in a Pet Dog Model of Diffuse Large B-cell Lymphoma", CLINICAL CANCER RESEARCH, vol. 20, no. 3, 3 December 2013 (2013-12-03), US, pages 668 - 677, XP055509931, ISSN: 1078-0432, DOI: 10.1158/1078-0432.CCR-13-2283
- DANIEL R. CIOCCA ET AL: "A pilot study with a therapeutic vaccine based on hydroxyapatite ceramic particles and self-antigens in cancer patients", CELL STRESS AND CHAPERONES, vol. 12, no. 1, 1 March 2007 (2007-03-01), pages 33 - 43, XP055131849, ISSN: 1355-8145, DOI: 10.1379/CSC-218R.1
- SRIVASTAVA: "IMMUNOTHERAPY OF HUMAN CANCER: LESSONS FROM MICE", NATURE IMMUNOLOGY, NATURE PUBLISHING GROUP US, NEW YORK, vol. 1, no. 5, 1 November 2000 (2000-11-01), pages 363 - 366, XP008130878, ISSN: 1529-2908, DOI: 10.1038/80795
- ROSSI JEAN-FRANCOIS ET AL: "Apavac, a Simple Autologous Vaccination Process (AV) Associated with Clinical Efficacy in 56 Patients (pts) Having Cancer (C) and 11 Pts Having Hematological Malignancies (HM)", BLOOD, vol. 132, no. Suppl. 1, 29 November 2018 (2018-11-29), & 60TH ANNUAL MEETING OF THE AMERICAN-SOCIETY-OF-HEMATOLOGY (ASH); SAN DIEGO, CA, USA; DECEMBER 01 -04, 2018, pages 5690, XP002790934

## Description

### Domaine technique de l'invention

La présente invention concerne le traitement du cancer par immunothérapie. La présente invention concerne particulièrement la production de vaccins autologues contre le cancer.

### Arrière-plan technologique

L'immunothérapie est une alternative thérapeutique reconnue et bien établie pour traiter le cancer. Elle regroupe plusieurs thérapies différentes, toutes basées sur la stimulation du système immunitaire du patient afin de reconnaitre et d'attaquer sa maladie.

Les cellules du système immunitaire sont normalement capables de surveiller et de détecter la présence d'anomalies au sein des cellules de l'organisme. La plupart des tumeurs développent cependant plusieurs mécanismes afin d'échapper à cette surveillance ; on observe alors une tolérance du système immunitaire envers la tumeur. Stimuler les cellules immunitaires, telles que les lymphocytes T, de manière à ce qu'elles reconnaissent spécifiquement les cellules tumorales permet de lever cette tolérance.

Cette stimulation peut, par exemple, être effectuée en mettant directement en contact (*in vitro* ou *in vivo*) des antigènes tumoraux avec les macrophages ou des cellules apparentées pour que les cellules présentatrices d'antigènes (APC) stimulent les lymphocytes T. C'est le concept d'un vaccin thérapeutique contre le cancer. Pour une stimulation in vivo, le principe est de prélever des protéines anormales de la tumeur et de les réinjecter sous une forme visible pour le système immunitaire.

Les vaccins thérapeutiques contre le cancer peuvent reposer sur l'emploi de protéines du choc thermique (HSPs) telles que la gp 96, ou l'HSP 70. Ces protéines sont des molécules chaperonnes et s'associent à des nombreux peptides dont les antigènes spécifiques de la tumeur de chaque patient. Elles constituent ainsi une empreinte moléculaire de la tumeur à éradiquer et diffèrent d'un patient à un autre et d'une tumeur à l'autre. Pour la même tumeur, cette dernière évolue dans le temps.

Cette stratégie de vaccination impose de purifier les protéines vaccinantes à partir de chaque tumeur contre laquelle elles doivent immuniser le patient et à un temps donné. Le protocole de purification est long, difficilement industrialisable et sujet à de multiples contaminations par endotoxines. Il est classique de purifier les HSPs à partir d'un broyât tumoral qui est soumis à une série de centrifugation, précipitation, chromatographie sur Con A, analyse électrophorétique, et chromatographie sur Mono Q FPLC. Les brevets US Nos 6,447,781, 6,436,404, 6,410,028, 6,383,494 et 6,030,618 décrivent des méthodes de purification de protéines HSP consistant à mettre en œuvre des colonnes chromatographiques de Con A SEPHAROSE.

Dans la demande de brevet WO2006/122914, il a été décrit l'utilité d'utiliser des particules d'hydroxyapatite (HAP) pour purifier les protéines vaccinantes à partir d'extraits tissulaires. Cette demande décrit plus particulièrement un procédé de préparation en une seule étape d'antigènes tumoraux destiné à les mettre sous une forme reconnaissable par le système immunitaire afin de pouvoir l'appliquer à grande échelle par un personnel n'ayant pas une qualification de biochimiste. WO 2006/122914 décrit en outre que les poudres d'HAP ainsi que d'autres sels de calcium peuvent être utilisés comme adjuvants de vaccination et que la poudre d'hydroxyapatite ayant adsorbé les antigènes tumoraux spécifiques d'une tumeur peut être utilisée en tant que médicament contre ladite tumeur. Ainsi, la même poudre d'HAP peut servir à la fois à purifier les protéines spécifiques d'une tumeur et stimuler le système immunitaire contre ces dernières lorsque poudres et protéines sont injectées ensembles.

Dans la demande de brevet WO2014/184453, il a en outre décrit le développement d'un procédé de production d'une poudre d'hydroxyapatite et/ou de phosphate tricalcique perfectionné. La poudre ainsi obtenue est mise en contact avec des protéines tumorales directement purifiées de biopsies tumorales, et donc marquant l'identité cellulaire de la tumeur, afin de produire un vaccin thérapeutique anti-tumoral augmentant le niveau d'immunité innée sans toxicité supplémentaire ni effet secondaire.

Cette technique est sûre, peut être associée à la chimiothérapie et ne produit aucun résidu toxique. Les inventeurs ont démontré que les lymphocytes T étaient capables de reconnaître les cellules tumorales après la vaccination. Le cycle de vaccination est très proche de celui utilisé pour le vaccin anti-infectieux. Le vaccin est injecté dans le tissu sous-cutané et plusieurs injections sont nécessaires (une par semaine pendant 4 semaines et une par mois pendant 4 mois).

Ce type d'immunothérapie peut faire la différence entre la rémission et la guérison de certains cancers à mauvais pronostic. Ceci a par exemple été démontré en médecine vétérinaire, où cette vaccination augmente la survie des chiens par rapport à la chimiothérapie seule pour un cancer mortel tel que les lymphomes B de haut grade (DLBCL). D'excellents résultats ont également été obtenus pour des tumeurs solides telles que les cancers des os (ostéosarcomes), les mastocytomes ou encore les mélanomes.

Cependant la production de ces vaccins à partir de biopsies présente plusieurs difficultés qui gênent considérablement une mise en œuvre industrielle. La première de ces difficultés est la nécessaire biopsie de la tumeur dans une zone qui ne soit pas nécrosée et qui soit représentative au niveau antigénique de l'ensemble de la tumeur et de ses éventuelles métastases.

D'autre part les biopsies ne peuvent pas toujours être obtenues, notamment si elles représentent par elles-mêmes un risque vital ou un risque de séquelles importantes chez des individus pour lesquels le risque anesthésique est important en raison des pathologies multiples et délabrantes. Ce risque est particulièrement important pour des tumeurs comme les adénocarcinomes du pancréas ou des tumeurs cérébrales difficilement accessibles ou même des tumeurs de la prostate de haut grade en raison des risques de dissémination.

De plus, sur un plan réglementaire, la gestion des biopsies est une affaire lourde et compliquée. Elle nécessite la création d'une banque de tissus, de suivre une réglementation stricte, d'assurer une surveillance de la chaine de froid et prendre en charge une logistique complexe.

Le coût d'une biopsie et de sa gestion dans une banque de tissu peut donc facilement être supérieur à celui du traitement proprement dit.

Il serait donc extrêmement intéressant et bénéfique de pouvoir développer un vaccin contre le cancer qui puisse être produit sans à avoir recours à des biopsies tumorales tout en conservant une efficacité comparable aux vaccins produits à partir de tumeurs.

### Brève description de l'invention

De manière surprenante, les présents inventeurs sont parvenus à développer un vaccin contre le cancer présentant des résultats comparables, voire supérieurs, à ceux obtenus avec le vaccin décrit dans la demande WO2014/184453 (basé sur des protéines issues de biopsies tumorales et des particules d'HAP), mais ce, sans avoir recours à une biopsie de tumeur.

Les présents inventeurs se sont en effet rendu compte qu'il était possible de développer un tel vaccin en extrayant directement les protéines tumorales à partir du sang, et notamment du sérum, d'un individu atteint de cancer.

Dans ce cadre la présente invention porte sur un procédé de préparation d'un vaccin autologue contre le cancer, ledit procédé comprenant les étapes suivantes :
a) extraction des protéines contenues dans un échantillon de sérum ou de plasma obtenu à partir d'un patient atteint de cancer ; et
b) mise en contact des protéines extraites à l'étape a) avec des particules d'hydroxyapatite et/ou de phosphate tricalcique.

La présente invention porte également sur le vaccin autologue obtenu à partir du procédé de préparation mentionné ci-dessus.

Enfin, la présente invention porte également le vaccin autologue obtenu à partir du procédé de préparation mentionné ci-dessus pour son utilisation en thérapie, et particulièrement dans le traitement du cancer chez le patient à partir duquel l'échantillon de sérum et ou plasma a été obtenu (vaccin autologue).

### Description détaillée de l'invention

Les présents inventeurs ont démontré qu'il était possible de développer un vaccin efficace contre le cancer basé sur des particules d'hydroxyapatite présentant des antigènes tumoraux, lesdits antigènes tumoraux ayant été obtenus non pas à partir de biopsies de tumeurs comme cela était le cas dans l'art antérieur, mais à partir d'échantillons de plasma ou de sérum du patient.

Dans un premier aspect, la présente invention porte donc sur un procédé de préparation de vaccin contre le cancer, et particulièrement d'un vaccin autologue contre le cancer, ledit procédé de préparation comprenant les étapes suivantes:
a) extraction des protéines contenues dans un échantillon de sérum ou de plasma obtenu à partir d'un patient atteint de cancer ; et
b) mise en contact des protéines extraites à l'étape a) avec des particules d'hydroxyapatite et/ou de phosphate tricalcique.

Un vaccin « autologue » désigne un vaccin dans lequel les protéines/antigènes tumoraux sont obtenus à partir du patient destiné à être vacciné.

Le « patient » ou « sujet » est un être humain ou un animal, par exemple un mammifère, et notamment le chien, le cheval ou le chat.

Un échantillon de sérum ou de plasma est typiquement obtenu depuis un échantillon de sang prélevé à partir du patient à traiter. Le sang est soumis à une centrifugation afin de séparer les composant les plus lourds du surnageant. Ce surnageant constitue le plasma s'il s'agit de sang anti-coagulé ou le sérum si le sang a coagulé naturellement.

La notion de « protéines/antigènes tumoraux » est bien connue de l'homme du métier. Il s'agit de protéines et/ou de molécules exprimées spécifiquement par les cellules tumorales et qui peuvent être reconnues par les lymphocytes T et B.

Selon la présente invention, les protéines/antigènes tumoraux sont directement extraits à partir de l'échantillon de sérum ou de plasma obtenu du patient à traiter. Typiquement la masse des protéines/antigènes tumoraux extraits à partir de l'échantillon de sérum ou de plasma est comprise entre 60 kDa et 130 kDa, en particulier entre 70 kDa et 110 kDa.

L'homme du métier connait un grand nombre de techniques permettant d'extraire des protéines depuis un échantillon de sérum ou de plasma. Typiquement les protéines/antigènes tumoraux sont extraites en précipitant l'échantillon de sérum dans une solution saline, le mélange ainsi obtenu est ensuite centrifugé, et le culot obtenu représente les protéines extraites.

Dans un mode de réalisation particulier, l'ensemble des protéines/antigènes extraits du plasma/sérum est utilisé et mis en contact pour préparer le vaccin selon la présente invention. C'est-à-dire que l'on met l'ensemble du « culot » de protéines/antigènes extraits du plasma/sérum en contact avec les particules d'hydroxyapatite.

L'« hydroxyapatite », est un minéral de la famille des phosphates de calcium, de formule Ca₁₀(PO4)₆(OH)₂, maille de la structure cristalline est hexagonale. L'hydroxyapatite est le membre hydroxylé du groupe apatite. Les ions de la maille cristalline sont substituables par d'autres ions de charges et de tailles prôches, il existe également des tunnels dans la maille qui peuvent accueillir des petites molécules comme certains acides aminés. Ce sont ces particularités qui donnent à ce minéral des propriétés d'adsorption très spécifiques.

Les particules d'hydroxyapatite selon la présente invention sont obtenues via le même procédé que celui décrit dans la demande WO2014/184453 dont le contenu est ici incorporé par référence. Cette document décrit notamment un procédé de production de particules d'hydroxyapatite phosphocalcique Ca₁₀(PO4)₆(OH)₂ consistant en une précipitation lente à haute température, obtenue par double décomposition d'un sel de calcium et d'un sel de phosphore en milieu basique. La réaction se passe à température constante dans un volume réactionnel important et est suivie d'une phase de maturation et d'une phase de lavage à l'eau. Le précipité ainsi obtenu subit une nouvelle étape de transformation : la séparation solide/solution. Cette dernière étape est réalisée soit par filtration, séchage par étuvage et concassage, soit par atomisation à l'aide d'un lit fluidisé. Quelle que soit la technique de séparation solide/solution choisie, la poudre va subir deux étapes de transformations spécifiques à l'application de l'invention : l'étape de sélection granulométrique par tamisage à sec pour ne retenir que la tranche granulométrique d'intérêt, inférieure à 25 µm ou comprise entre 25 et 45 µm, puis une dernière étape durant laquelle la poudre sera frittée à une température optimale pour assurer une fusion des grains conduisant à des états de surface des poudres bien particuliers (préférentiellement supérieur ou égal à 30m²/g). Ces deux dernières étapes peuvent être inversées : sélection puis frittage ou frittage puis sélection. Le "frittage", est un procédé consistant à chauffer une poudre sans la mener jusqu'à la fusion. Dans le cadre de la présente invention, le frittage est par exemple réalisé à une température comprise entre 400°C et 600°C. L'HAP ainsi obtenue peut être sous forme de poudre et subir un ou plusieurs lavages.

Typiquement, les protéines/antigènes tumoraux sont mis en contact avec des particules d'hydroxyapatite en faisant passer lesdits protéines/antigènes tumoraux sur une colonne de particules d'hydroxyapatite, telle qu'une colonne de chromatographie. Les antigènes tumoraux peuvent éventuellement être mis en contact en solution puis lavés par centrifugation. Les protéines/antigènes tumoraux sont alors adsorbées sur la surface des particules d'hydroxyapatite. Les particules d'hydroxyapatite peuvent particulièrement être sous forme de poudre.

Lorsqu'une colonne de chromatographie est utilisée elle peut, par exemple être mise sous pression.

Une fois les particules d'hydroxyapatite chargées en protéines/antigènes tumoraux, elles sont mises en suspension dans un liquide d'injection et injectées au patient à partir duquel l'échantillon de sérum/plasma a été obtenu.

Typiquement, le liquide d'injection comprend un agent organique facilitant l'injection tel que la carboxymethylcellulose.

Un aspect de l'invention porte sur un vaccin autologue comprenant des particules d'hydroxyapatite et/ou de phosphate tricalcique chargées en antigènes tumoraux obtenus à partir d'un échantillon de sérum ou de plasma d'un patient atteint d'un cancer.

Typiquement, le vaccin autologue est sous la forme d'une suspension.

Typiquement, la masse des antigènes tumoraux extraits à partir de l'échantillon de sérum ou de plasma est comprise entre 60 kDa et 130 kDa, en particulier entre 70 kDa et 110 kDa.

Selon un mode de réalisation particulier, le vaccin autologue est obtenu selon le procédé décrit ci-dessus.

Le vaccin autologue selon la présente invention comprend donc les particules d'HAP et/ou de phosphate tricalcique ayant adsorbé les antigènes tumoraux spécifiques du patient (obtenus à partir d'un échantillon de sérum ou de plasma dudit patient) et ayant été remises en suspension.

Ce vaccin est ensuite administré de préférence par injection, par exemple par injection sous-cutanée ou intradermique. Le vaccin peut être administré par voie orale ou par tout autre moyen permettant une vaccination transmucosale.

La présente invention porte également sur l'utilisation du vaccin autologue obtenu selon le procédé ci-dessus pour son utilisation en thérapie, et particulièrement dans le traitement du cancer chez le patient à partir duquel les protéines/antigènes tumoraux ont été obtenus.

Un autre aspect de l'invention porte en outre sur une méthode de traitement d'un patient souffrant d'un cancer, ladite méthode comprenant les étapes suivantes :
a) extraction de protéines contenues dans un échantillon de sérum ou de plasma obtenu à partir d'un patient atteint de cancer ;
b) mise en contact des protéines extraites à l'étape a) avec des particules d'hydroxyapatite et/ou de phosphate tricalcique ;
c) mise en suspension dans un liquide d'injection des particules d'hydroxyapatite et/ou de phosphate tricalcique mises en contact avec les protéines à l'étape b),
d) injection du mélange obtenu à l'étape c) au patient à partir duquel l'échantillon de sérum ou de plasma utilisé à l'étape a) a été obtenu.

Dans le cadre de la présente invention, le « cancer » peut être n'importe quel cancer pour lequel une immunothérapie peut être indiquée. Le cancer peut particulièrement être choisi parmi une liste non exhaustive comprenant les mélanomes, les carcinomes ou adénocarcinomes qui se développent à partir des cellules épithéliales et/ou de cellules d'une glande, les sarcomes se développant à partir des cellules des tissus conjonctifs ou musculaires, les tumeurs du système nerveux central, les tumeurs du système hématopoïétique comme les leucémies, et lymphomes. On peut également inclure les différents cancers d'origine infectieuse dont les plus représentatifs sont les cancers du col, le cancer primitif du foie et les cancers gastriques.

Dans un mode de réalisation particulier, le cancer est choisi dans le groupe constitué des ostéosarcomes, des lymphomes B ou T, des tumeurs mammaires, des mélanomes, des hémangio-sarcomes, des mastocytomes, des fibrosarcomes, des tumeurs cérébrales ou du système nerveux central, des schwanomes, des mésothéliomes, des séminomes, des tératomes et des blastomes.

Dans un mode de réalisation préféré, le cancer est choisi parmi un glioblastome, un sarcome (tel que l'ostéosarcome ou le fibrosarcome), un mélanome, un carcinome ou un adénocarcinome.

Dans un mode de réalisation particulier, le cancer est un cancer disséminé, c'est-à-dire présentant des métastases (patients classés dans la catégorie NxMx selon la classification TNM).

Le patient peut recevoir une à plusieurs injections, doses de vaccin. Une dose comprend généralement entre 30 et 50 mg d'hydroxyapatite et/ou de phosphate tricalcique et entre 1000 et 2000 µg de protéines.

De manière préférée, le patient peut recevoir plusieurs injections espacées dans le temps, par exemple de plusieurs jours, semaines ou mois. Dans un mode de réalisation préféré, les injections sont séparées d'une semaine pendant le premier mois puis d'un mois pendant 4 mois.

Chaque injection peut être idéalement préparée à partir d'un nouveau prélèvement de protéines tumorales chez le patient en cas de signe d'échappement au vaccin ou d'inefficacité en particulier lors d'augmentation du taux de marqueurs tumoraux.

Le présent vaccin représente une évolution considérable du protocole par rapport à la technique mettant en œuvre des biopsies tumorales : les protéines n'ont pas besoin d'être extraites du secteur intra-cytoplasmique.

Les électrophorèses SDS Page des protéines fixées sur les poudres prêtes à être injectées montrent que les bandes protéiques sont différentes de celles obtenues par biopsie tumorale (comme dans la demande WO2014/184453). De plus comme démontré ci-après, les résultats obtenus avec un vaccin selon la présente invention sont aussi bon, voir meilleurs.

Le vaccin autologue selon la présente invention peut être utilisé en combinaison avec un autre traitement anticancéreux tel que la radiothérapie, la chimiothérapie ou un autre agent immunothérapeutique.

La présente invention est présentée de manière plus détaillée dans les exemples ci-dessous. Ces exemples sont fournis uniquement à des fins illustratives et ne peuvent être interprétés comme limitant la portée de la présente invention.

### Brève description des Figures

**Fig 1** : SDS Page de protéines détachées de la surface de la poudre de vaccins fabriqués à partir de différentes tumeurs. De gauche à droite : adenocarcinome mammaire, lymphome diffus, et ostéosarcome. Les résultats sont variables tant qualitativement que quantitativement. Echelle en KDa.
**Fig. 2** : SDS Page de protéines obtenues à partir de vaccins fait à partir du sérum. De gauche à droite, mélanome (deux première colonnes), cancer de la langue (2 suivantes), hémangiosarcome (2 suivantes). Echelle en KDa. Il y a beaucoup moins de contamination, les principales bandes quantitativement se trouvent entre 160 et 110 kDa. Ceci est très reproductible.
**Fig 3** **:** marquage d'une coupe de glioblastome par des anti IgG humains montrant que des anticorps anti cellules tumorales sont présents après vaccination comme le montre le marquage par la péroxydase (dépôt brun).

### EXEMPLES

Le présent protocole a été effectué chez des patients manifestant des pathologies à très mauvais pronostic, non curables par les thérapies alors utilisées. Les patients ont participé au présent protocole en en faisant la demande expresse et en signant un consentement éclairé.

Le présent protocole peut être indiqué pour les cancers disséminés en dernière ligne après l'échec des thérapies classiques associé ou non à une chimiothérapie ou une autre immunothérapie. La prévention de récidives pour des cancers sans thérapie efficace après chirurgie peut également être envisagée.

Vingt-sept patients ont été inclus dans ce protocole. Neuf patients ont été soumis au protocole d'immunothérapie sans aucun traitement de chimiothérapie ajouté. 10 patients avaient un glioblastome, 2 un sarcome et 15 un carcinome ou un adénocarcinome. 22 patients avaient une maladie évolutive lors de l'application du protocole d'immunothérapie, 13 de ces patients étaient indemnes de maladie ou avec une maladie stable ou une régression partielle 5 mois après le début du protocole. Lorsque les glioblastomes ont été mis de côté, 72% des patients qui étaient atteints de maladie évolutive au moment de la vaccination sont passés deux mois après la vaccination en état stable (SD) ou en régression partielle (PR). Dans les conclusions bien que cette série soit hétérogène, un pourcentage important de cas ont montré une réponse clinique à cette technique de dernière ligne et aucun effet secondaire n'est survenu. Les cancers disséminés peuvent être stabilisés par cette technique montrant que la réponse immunitaire peut être obtenue chez l'homme, même pour une maladie avancée et évolutive rapide.

### EXEMPLE 1 : Différence entre l'électrophorèse des protéines adsorbées sur les poudres en contact avec le sérum ou avec une biopsie tumorale.

Les électrophorèses des protéines contenues dans les vaccins effectués à partir d'une biopsie ou bien à partir des protéines sériques ont été comparées.

Les particules d'HAP ont typiquement été obtenues selon le procédé décrit dans l'Exemple 1 de la demande WO2014/184453.

Les doses ont été préparées comme il suit :

### - A partir d'une biopsie :

Le tissu tumoral et tous les matériaux utilisés pour préparer le vaccin ont été manipulés stérilement sous une hotte à flux laminaire. Le tissu tumoral congelé (200 mg) a été homogénéisé en utilisant un homogénéisateur de tissu à billes. 1 ml de NaHCO3 (30 mM, pH 7) a été ajouté pour 1 ml d'homogénat.

L'homogénat résultant a été ensuite centrifugé à 1000 g pendant 15 mn à 4 ° C pour éliminer toutes les débris tissulaires. Le surnageant est mélangé à 50% dans une solution sursaturée en nitrate d'ammoniaque et placée une heure à 4°C puis centrifugé. Le culot est remis en suspension dans un tampon phosphate 0,02M, pH7. Cette solution est ensuite percolée dans une colonne de chromatographie contenant la poudre d'HAP. La colonne a été remplie (colonnes de chromatographie, Poly-prep, Cat.731-1550, Bio Rad) avec 0,2 gr de HAP (0-25 µm), équilibré avec 10 volumes de tampon phosphate (20 mM pH7). Le culot remis en suspension a ensuite été ajouté. La colonne a ensuite été lavée avec 3 ml d'une solution de NaCl 100 mM. La poudre a ensuite été mise en suspension dans 5 ml de solution de carboxyméthylcellulose (CMC) (2% dans 20 mM NaCl). 0,5 ml de cette solution a été utilisé pour chaque injection de vaccin.

### - A partir du sérum :

La procédure est beaucoup plus simple, 3cc de sérum sont dilués à 50% dans une solution de nitrate d'amoniaque sursaturée, placée à 4°C une heure puis centrifugée. Le culot est remis en suspension dans un tampon phosphate 0,02M, pH7. Cette solution est ensuite percolées dans une colonne de chromatographie contenant la poudre d'HAP. La colonne a été remplie (colonnes de chromatographie, Poly-prep, Cat.731-1550, Bio Rad) avec 0,2 gr de HAP (0-25 µm), équilibré avec 10 volumes de tampon phosphate (20 mM pH7). Le culot remis en suspension a ensuite été ajouté. La colonne a ensuite été lavée avec 3 ml d'une solution de NaCl 100 mM. La poudre a ensuite été mise en suspension dans 5 ml de solution de carboxyméthylcellulose (CMC) (2% dans 20 mM NaCl). 0,5 ml de cette solution a été utilisé pour chaque injection de vaccin.

Dans les deux cas pour l'électrophorèse, les protéines adsorbées sur les poudres sont désorbées avant l'électrophorèse. 10 mg de poudres d'une dose vaccinale sont centrifugées deux minutes à 500 g. Le surnageant est éliminée le culot est remis en suspension dans 0,5 ml de de NaCl 0.5M. Les poudres sont agitées remises en suspension et centrifugées à 500 g. 10 µl du liquide surnageant sont ensuite mis dans les puits d'électrophorése après avoir été dilué à 50% dans du détergent et chauffé à 70°C pendant 5 mn.

### Résultats :

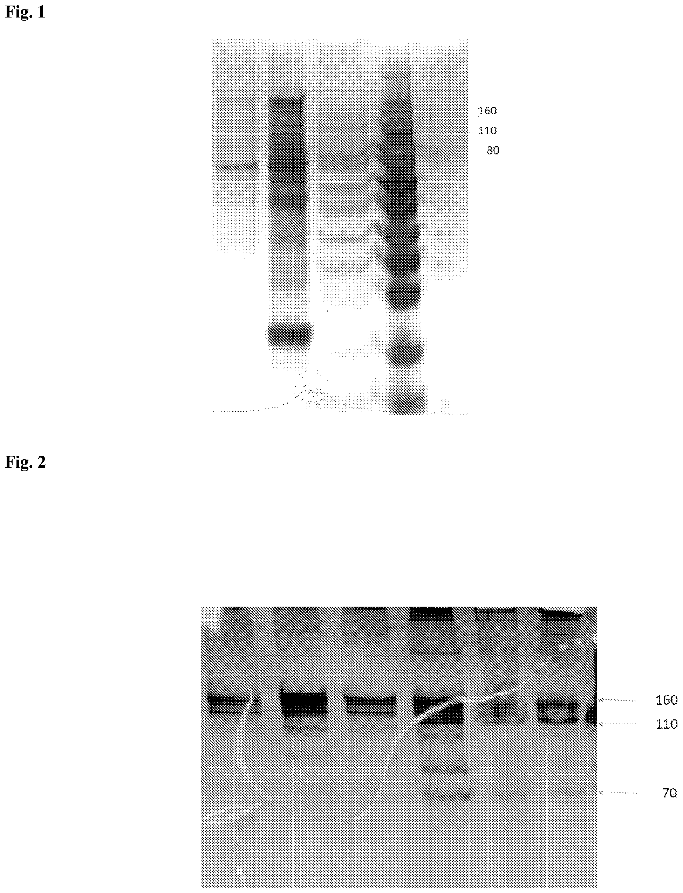

Il existe des différences notables entre les profils électrophorétiques obtenus par les deux méthodes. Les plus marquantes sont une diminution du nombre de bandes sur les électophorèses provenant du sang par rapport à celle provenant de biopsies (fig. 1, 2). Les électrophorèses provenant du sang sont également beaucoup plus homogènes montrant une reproductibilité meilleure de la purification.

### EXEMPLE 2 : Résultats de l'administration du vaccin selon l'invention chez une patiente souffrant d'adénocarcinome

Cette patiente a présenté un adénocarcinome de l'endomètre (TNM 7a) traité par deux cycles de paclitaxel et de carboplatine pré et post opératoire. 18 mois après que la patiente a manifesté une miliaire pulmonaire et des localisations secondaires sur des sites rétropéritonéaux, elle a reçu un traitement hormonal (acétate de mégestrol) et commence les vaccins. A trois mois (IRM, TEP) après vaccination, elle a montré une stabilisation de sa pathologie pulmonaire et une légère régression du volume des masses rétropéritonéales, qui s'est ensuite poursuivie puisqu'à 18 mois les masses rétropéritonéales ont presque disparu et la miliaire pulmonaire toujours présente avait régressé. Cette régression partielle est toujours stable après deux ans et demi d'observation.

Cet exemple montre que l'effet clinique de la vaccination à partir des protéines sériques peut être maintenu sur un temps important supérieur à 30 mois.

### EXEMPLE 3 : Résultats de l'administration du vaccin selon l'invention chez un patient souffrant d'adénocarcinome du colon

Ce patient a présenté un adénocarcinome du colon traité chirurgicalement et par adriamycine. A trois ans, des métastases pulmonaires et hépatiques sont apparues localisées dans un lobe pulmonaire et un lobe hépatique ce qui a permis une chirurgie du poumon et du foie suivies de chimiothérapie. A 5 ans, une chimiothérapie par gemcitabine, capecitabine, bevacizumab a été instituée après une réapparition de nombreuses métastases hépatiques et ganglionnaires retropéritonéales. Malgré la chimiothérapie, la maladie est restée en progression. A la suite d'un arrêt de la chimiothérapie suite à une embolie pulmonaire, les marqueurs tumoraux (ACE) ont fortement augmenté. Le patient a été traité par vaccination à partir des protéines sériques (4 injections à une semaine d'intervalle le premier mois puis une injection/moisentraînant une baisse marquée du taux de marqueur. Les marqueurs sont ensuite remontés 3 mois après suggérant un échappement de la tumeur au vaccin avec un PET (tomographie par émission de positrons) identique à celui fait avant la vaccination. Une autre préparation a donc été faite à partir d'un autre prélèvement de sérum et injectée toutes les trois semaines, ce qui a eu pour conséquences de faire baisser les marqueurs en dessous des valeurs normales en moins de trois mois. Le PET fait état de nécroses des métastases hépatiques un an après le début de l'immunothérapie.

En conclusion: le vaccin fait à partir du sang inhibe la prolifération cellulaire dans un premier temps détectable par les marqueurs de prolifération cellulaires puis manifeste un effet plus tardif détectable par PET sur un temps beaucoup plus long de plusieurs mois à un an.

### EXEMPLE 4 : Anticorps sérique anti glioblastome post vaccination avec vaccin préparé à partir du sang

Ce patient a été vacciné 6 mois après la découverte et la biopsie chirurgicale d'un glioblastoma de stade IV temporal droit. Le traitement de 1^{ère} ligne a été un protocole de Stupp (Stupp, R. et coll Radiotherapy plus Concomitant and Adjuvant Temozolomide for Glioblastoma, N Engl J Med 2005; 352:987-996) à base de témodal alterné avec de la radiothérapie. La vaccination à partir des protéines sériques a été menée à une fréquence de 4 injections à une semaine d'intervalle pendant un mois puis une injection par mois tout le temps de la survie du patient. Le patient est resté en rémission complète pendant 10 mois en en progression (critères RECIST) pendant 2 mois avant de décéder. Nous avons testé la présence d'anticorps sériques (IgG) sur des coupes de la biopsie tumorale du patient afin de voir si des anticorps contre les cellules tumorales existaient.

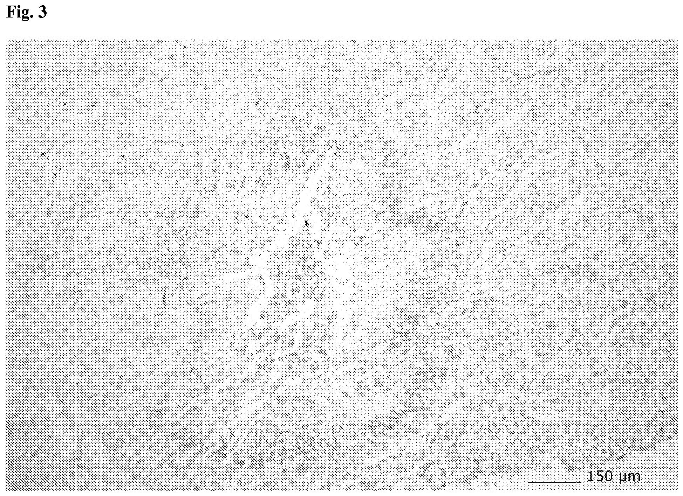

Des coupes de tissu de 5 µm d'épaisseur incluses dans la paraffine sont utilisées. Ils sont déparaffinés dans du xylol et réhydratés dans des solutions réductrices d'éthanol. Les peroxydases endogènes sont inhibées par une solution de H₂O₂. Le sérum du patient est dilué à 1/100 dans un tampon de Hepes et sert d'anticorps primaire dans un marqueur en utilisant un anti-IgG humain marqué à la peroxydase comme anticorps secondaire. La peroxydase est ensuite démontrée par du DAB (3,3'-diaminobenzidine) oxydé en présence de peroxyde d'hydrogène peroxydase produisant un dépôt brun insoluble dans l'alcool (Figure 3). Le témoin négatif est une coupe traitée dans les mêmes conditions sans l'anticorps primaire (sérum dilué).

Le marquage montre qu'une majorité des cellules tumorales est positive mais il faut noter que certaines d'entre elles ne le sont pas. Ceci suggère que le vaccin provoque une réaction contre les cellules tumorales et que la période de temps existant entre la biopsie et la fabrication du vaccin a fait que tous les clones ne sont pas représentés dans le vaccin.

Cette histologie montre que la vaccination par les protéines sériques déclenche une réaction immunitaire anti tumorale humorale.

### EXEMPLE 5: Différence entre le pourcentage d'ELISPOT positifs induits par les vaccins fabriqués à partir d'une biopsie tumorale et ceux fabriqués à partir du sérum.

Nous avons comparé la stimulation des CD8 induite par les vaccins fabriqués à partir du sang ou à partir d'une biopsie. Nous avons une série de patients avec diverses pathologies cancéreuses pour lesquelles une biopsie ou un prélèvement de sérum a été fait en fonction de la disponibilité des biopsies. Il faut noter que la stimulation de l'immunité cellulaire détectée par Elispot n'est pas toujours corrélée avec le résultat clinique. L'Elispot ne détecte pas tous les états de stimulation des CD8. Le test ELISPOT a été fait chez chaque patient à la cinquième injection c'est-à-dire trois mois après la première vaccination.

10 ml de sang sont recueillis dans un tube citraté et centrifugés dans les 4 heures après prélèvement dans du ficoll 400. Les cellules nucléées sont ensuite lavées dans du milieu RPMI et mise en culture dans des puits coatés par des protéines vaccinales à une concentration de 2. 10⁵ cellules/puits. Les protéines vaccinales sont obtenues comme il a été décrit précédemment pour faire les électrophorèses des vaccins. 10 mg de poudres chargées en protéines sont lavés dans 0,5 ml de NaCl 0,2M. Pour enduire les boîtes la solution est ramenée à 0,02M. Les cellules sont ensuite cultivées toute une nuit dans du milieu RPMI supplémenté en 5% de sérum de veau fœtal à 37°C dans un incubateur à 5% de CO₂. Les cellules sont ensuite lavées 10 mn dans du PBS contenant 0,1% de détergent tween20. Les puits sont ensuite incubés une heure dans un ml de PBS avec 0,1% de BSA et l'anticorps anti interféron gamma marqué à la peroxydase dilué au 1/1000. La péroxydase est ensuite mise en évidence comme dans l'exemple 4.

15 patients ont été vaccinés par des vaccins faits à partir du sérum et 13 ont été vaccinés à partir de vaccins faits à partir de biopsies tumorales. Les ELISPOT ont été effectués au moment de la 5^{éme} injection c'est-à-dire au troisième mois de vaccination. Le témoin négatif est le sang d'un patient non vacciné ayant une tumeur de même type. 14/15 patients vaccinés à partir du sérum ont des ELISPOT positifs alors que seulement 6/13 des patients vaccinés à partir des biopsies ont un ELISPOT positif. Ce qui constitue une différence statistiquement différente.

Au vu de ces différents résultats, compte tenu des différences de composition visibles par électrophorèse et des résultats constatés tant cliniquement que biologiquement, il existe un effet surprenant du vaccin obtenu par cette méthode.

## Revendications

1. Procédé de préparation d'un vaccin autologue contre le cancer comprenant des particules d'hydroxyapatite et/ou de phosphate tricalcique chargées en antigènes tumoraux, ledit procédé comprenant les étapes suivantes :
a) extraction des protéines contenues dans un échantillon de sérum ou de plasma obtenu à partir d'un patient atteint de cancer ; et
b) mise en contact des protéines extraites à l'étape a) avec des particules d'hydroxyapatite et/ou de phosphate tricalcique afin d'obtenir le vaccin autologue.

2. Procédé selon la revendication 1, dans lequel le patient est un humain.

3. Procédé selon la revendication 1, dans lequel le patient est un chien, un cheval ou un chat.

4. Procédé selon l'une quelconque des revendications 1 à 3, dans lequel le cancer est choisi parmi les mélanomes, les carcinomes, adénocarcinomes, les sarcomes, les tumeurs du système nerveux central, les leucémies, les lymphomes et les cancers d'origine infectieuse.

5. Procédé selon l'une quelconque des revendications 1 à 3, dans lequel le cancer est choisi dans le groupe constitué des ostéosarcomes, des lymphomes B ou T, des tumeurs mammaires, des mélanomes, des hémangio-sarcomes, des mastocytomes, des fibrosarcomes, des tumeurs cérébrales ou du système nerveux central, des schwanomes, des mésothéliomes, des séminomes, des tératomes et des blastomes.

6. Procédé selon l'une quelconque des revendications 1 à 3, dans lequel le cancer est un glioblastome, un sarcome, un mélanome, un carcinome ou un adénocarcinome.

7. Procédé selon l'une quelconque des revendications 1 à 6, dans lequel le cancer est un cancer présentant des métastases.

8. Vaccin comprenant des particules d'hydroxyapatite et/ou de phosphate tricalcique chargées en antigènes tumoraux **caractérisé en ce que** le vaccin est obtenu à partir d'un procédé de préparation selon l'une quelconque des revendications 1 à 7.

9. Vaccin selon la revendication 8 pour son utilisation dans le traitement du cancer chez le patient à partir duquel l'échantillon de sérum et ou plasma a été obtenu.

## Patentansprüche

1. Verfahren zur Herstellung eines autologen Impfstoffs gegen Krebs, der mit Tumorantigenen beladene Hydroxylapatit- und/oder Tricalciumphosphatpartikel umfasst, wobei das Verfahren die folgenden Schritte umfasst:
a) Extraktion der Proteine, die in einer Serum- oder Plasmaprobe enthalten sind, die von einem Krebspatienten erhalten wurde; und
b) Inkontaktbringen der in Schritt a) extrahierten Proteine mit Hydroxylapatit- und/oder Tricalciumphosphatpartikeln, um den autologen Impfstoff zu erhalten.

2. Verfahren nach Anspruch 1, wobei der Patient ein Mensch ist.

3. Verfahren nach Anspruch 1, wobei der Patient ein Hund, ein Pferd oder eine Katze ist.

4. Verfahren nach einem der Ansprüche 1 bis 3, wobei der Krebs gewählt ist aus Melanomen, Karzinomen, Adenokarzinomen, Sarkomen, Tumoren des Zentralnervensystems, Leukämien, Lymphomen und Krebserkrankungen infektiösen Ursprungs.

5. Verfahren nach einem der Ansprüche 1 bis 3, wobei der Krebs gewählt ist aus der Gruppe, umfassend Osteosarkome, B- oder T-Lymphome, Mammatumoren, Melanome, Hämangiosarkome, Mastozytome, Fibrosarkome, Hirntumoren oder Tumoren des Zentralnervensystems, Schwannome, Mesotheliome, Seminome, Teratome und Blastome.

6. Verfahren nach einem der Ansprüche 1 bis 3, wobei der Krebs ein Glioblastom, Sarkom, Melanom, Karzinom oder Adenokarzinom ist.

7. Verfahren nach einem der Ansprüche 1 bis 6, wobei der Krebs ein Krebs ist, der Metastasen aufweist.

8. Impfstoff, der mit Tumorantigenen beladene Hydroxylapatit- und/oder Tricalciumphosphatpartikel umfasst, **dadurch gekennzeichnet, dass** der Impfstoff aus einem Herstellungsverfahren gemäß einem der Ansprüche 1 bis 7 erhalten wird.

9. Impfstoff nach Anspruch 8 zur Verwendung bei der Behandlung von Krebs bei dem Patienten, von dem die Serum- und/oder Plasmaprobe erhalten wurde.

## Claims

1. A method for producing an autologous cancer vaccine comprising particles of hydroxyapatite and/or tricalcium phosphate loaded with tumor antigens, said method comprising the following steps:
a) extracting the proteins contained in a serum or plasma sample obtained from a patient suffering from cancer; and
b) bringing the proteins extracted in step a) into contact with particles of hydroxyapatite and/or of tricalcium phosphate in order to obtain the autologous vaccine.

2. The method as claimed in claim 1, wherein the patient is a human.

3. The method as claimed in claim 1, wherein the patient is a dog, a horse or a cat.

4. The method as claimed in any one of claims 1 to 3, wherein the cancer is chosen from melanomas, carcinomas, adenocarcinomas, sarcomas, central nervous system tumors, leukemias, lymphomas and cancers of infectious origin.

5. The method as claimed in any one of claims 1 to 3, wherein the cancer is chosen from the group consisting of osteosarcomas, B or T lymphomas, mammary tumors, melanomas, hemangio-sarcomas, mastocytomas, fibrosarcomas, brain or central nervous system tumors, schwannomas, mesotheliomas, seminomas, teratomas and blastomas.

6. The method as claimed in any one of claims 1 to 3, wherein the cancer is a glioblastoma, a sarcoma, a melanoma, a carcinoma or an adenocarcinoma.

7. The method as claimed in any one of claims 1 to 6, wherein the cancer is a cancer exhibiting metastases.

8. A vaccine comprising particles of hydroxyapatite and/or of tricalcium phosphate loaded with tumor antigens, **characterized in that** the vaccine is obtained from a production method as claimed in any one of claims 1 to 7.

9. The vaccine as claimed in claim 8, for use in the treatment of cancer in the patient from whom the serum and/or plasma sample was obtained.
